(19) Europäisches Patentamt
European Patent Office
Office européen des brevets

(11) **EP 2 696 127 A1**

(12) **EUROPEAN PATENT APPLICATION**
published in accordance with Art. 153(4) EPC

(43) Date of publication:
**12.02.2014 Bulletin 2014/07**

(21) Application number: **12842693.9**

(22) Date of filing: **20.08.2012**

(51) Int Cl.:
*F17C 13/02* (2006.01)     *F17C 5/00* (2006.01)
*B60K 15/06* (2006.01)

(86) International application number:
**PCT/KR2012/006589**

(87) International publication number:
**WO 2014/003234 (03.01.2014 Gazette 2014/01)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.06.2012  KR 20120068308**

(71) Applicant: **Gasroad, Co., Ltd
Yuseong-gu
Daejeon 305-500 (KR)**

(72) Inventors:
• **GIL, Young Man
Daejeon 305-755 (KR)**
• **KIM, Gwang Yun
Daejeon 305-306 (KR)**

(74) Representative: **Gille Hrabal
Brucknerstrasse 20
40593 Düsseldorf (DE)**

(54) **SYSTEM AND METHOD FOR MEASURING CHARGE AMOUNT OF PRESSURE VESSEL USING PRESSURE AND VOLUME**

(57)     A measuring system for the charged amounts in a pressure container using pressure and volume comprising: an information acquisition device that can collect information on volume and maximum charging pressure of the pressure container; first and second automatic valves, a reference container, a pressure sensor and a temperature sensor that are connected to a gas line between a storage tank and a charging nozzle; a control part that calculates the charged amounts through engineering calculations with pressure detected by the pressure sensor, temperature detected by the temperature sensor, and basic information on the pressure container and that calculates changes in temperature and volume of the pressure container to calibrate for the charged amounts, determine safety of the pressure container, and convert the calibrated charged amount to a sum of money; and a display part that receives the calculated information from the control part and inform it to a user.

FIG. 3

## Description

TECHNICAL FIELD

The present invention is related to a gas charging technique for charging gas in a pressure container, and to a measuring system and method for charged gas without using a flow meter but through measuring gas pressure and temperature of the system before and after charging, calculating the charged amount in the pressure container to determine safety of the pressure container, and converting the charged amount into a cost to inform the user.

TECHNICAL BACKGROUND

For types of gas fuels, compressed natural gas (CNG), liquefied natural gas (LNG), hydrogen, and liquefied petroleum gas (LPG), etc. are generally known. CNG has a richer reserve than other fuels and has a high octane value and yet has a low carbon dioxide emission coupled with a high combustion efficiency so that it is generally used as a fuel for vehicles along with liquid fuels. Hydrogen has also seen increased use as a next-generation alternative fuel with expanded development of fuel cell vehicles.

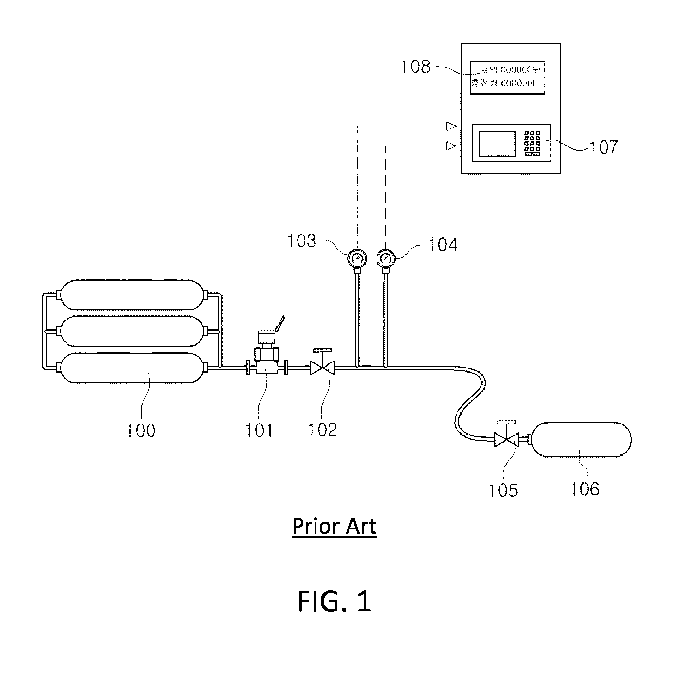

As shown in FIG. 1, such gas fuels are charged by a charging system where fuel gas stored in a storage tank (100) is supplied to a pressure container (106) through a mass flow meter (101), and the charged amount for fuel gas measured by the mass flow meter (101) is displayed by a display part (108) in the charged amount and the sum of money converted by a control part (107). Generally employed in the case of a vehicle charging system using compressed natural gas are a fixed type of system where a low-pressure (0.4 MPa) CNG is charged using a charger after being compressed to a high pressure (25 MPa), and a mobile type of system where it is connected with a charger for supplying to a vehicle after being charged from a tube trailer mounted with a high-pressure gas container at a fixed type of charging station and moved to a charging station such as garage, etc. Also a small-scale charging system composed simply of a compressor, safety device, and charger without being equipped with a storage container is also widely used.

As described above, for a charger equipping the fuel gas charging system, a Coriolis mass flow meter (101) which measures flux generally by sensing Coriolis force generated upon passage of gas is mainly used. Since such a mass flow meter is expensive, accounting for about a half of usual production cost of a charger, excessive costs are required to equip the charger.

DETAILED DESCRIPTION OF INVENTION

TECHNICAL PROBLEM

A purpose of the present invention is to solve the problems, namely, to provide a measuring system and method using gas pressure, temperature and a volume of the pressure container for fuels that are measured upon charging a gas fuel in the pressure container, without using a high-priced mass flow meter, to calculate the charged amount and to inform a user of an accurate charged amount through calibration of volume changes for the pressure container so as to save the production cost of a charger, to improve accuracy of the charged amount of gas fuel charged in the pressure container for preventing overcharging and to determine safety of the pressure container by identifying changes in the charged gas amount in the pressure container.

MEANS FOR SOLVING THE PROBLEM

A measuring system of the charged amount for the pressure system according to the present invention is equipped with first and second automatic valves, a reference container, a pressure sensor and a temperature sensor that are connected to a gas line between a gas fuel storage tank and a charging nozzle; with a control part that calculates the charged amount of gas fuel charged in the pressure container through engineering calculations with inputs such as pressure detected by the pressure sensor, temperature detected by the temperature sensor and volume information of the pressure container for charging of the gas fuel as well as changes in temperature and volume of the pressure container to calibrate for the charged amount and that converts the calibrated charged amount to the sum of money; and with a display part informing the user of the charged amount, the pressure and the sum of money provided by the above control part.

A measuring method for the charged amount in the pressure container using pressure and volume according to the present invention does not use a mass flow meter in the measuring method for the charged amount of fuel gas, but rather calculates the charged amount by measuring pressure and temperature of the charged fuel gas along with inputting or measuring of volume of the pressure container.

Stepwise composition is realized including a detection step for initial pressure and temperature values of the reference container that supplies gas fuel of the storage tank to the reference container by opening the first automatic valve while the second automatic valve is closed, and then closing the first automatic valve after detecting pressure and temperature of the reference container and transmitting them to the control part; a collection step for basic information of the subject pressure container for supplying gas fuel and a setting step for charging target values for the gas fuel charged in the pressure container; a detection step for pressure and temperature values before charging of the pressure container that, by opening the second automatic valve, forms identical pressure and temperature for reference and pressure containers, detecting the pressure and the temperature in this condition and transmits them to the control part; a determination step for charging implementation status that determines conducting status of the subsequent step to find a case and a volume to start charging depending on the acquisition status of volume information for the pressure container; an acquisition step of volume information for the pressure container that closes the second automatic valve and opens the first automatic valve followed by measuring pressure and temperature, and transmitting to the control part to calculate the volume of the pressure container and that forms the equalized pressures for reference and pressure containers by closing the first automatic valve and opening the second automatic valve, measures pressure and temperature and transmits them to the control part to calculate volume of the pressure container; a charging step for charging the gas fuel from the storage tank to the pressure container by opening the first automatic valve after acquiring volume information of the pressure container; a detection step for pressure and temperature values after charging the pressure container that closes the first automatic valve and detects pressure and temperature to be transmitted to the control part when the charged amount reaches a target charging value and equalized pressure and temperature conditions are achieved for gases in the pressure container and the reference container; a charging completion step where the charging nozzle is separated from the pressure container; a calculation step for the charged amount of the pressure container where the calibrated amount of fuel gas is calculated according to the amount of gas fuel that moved from the reference container to the pressure container at the detection step for pressure and temperature before charging in addition to the difference in volume of the gas fuel between before and after charging calculated by the above control part along with the changes in temperature or volume of the pressure container to calculate the charged amount for the pressure container, which is compared with the supplied amount to determine the exceeding status beyond the set value; and an informing step where agreement status of the charged amount and the set value is calculated for the pressure container to inspect the conditions of the pressure container and to inform the display part of the sum of money converted from the charged amount along with the safety through text or voice.

EFFECTS OF INVENTION

The present invention enables great savings in construction costs for a charging system by calculating the charged amounts through sensing pressure and temperature of the charged pressure container without using a conventional, high-priced mass flow meter.

The present invention produces an effect of improving precision for measurements of charged amounts even if the surrounding environment of the pressure container may be changed by outside temperatures, etc. through calculation of the rates of volume change according to the volume changes of the pressure container equipped in a system.

The present invention generates a time-saving effect for safety affirmation and inspection of the pressure container, since leakage or volume change, etc. may be inspected simultaneously with charging of fuel gas.

BRIEF DESCRIPTION OF THE DRAWINGS

FIG. 1 shows a charging system that employs a Coriolis mass flow meter according to conventional technology.
FIG. 2 shows a system for measuring the charged amounts of a pressure container using pressure and volume according to an example for implementing the present invention.
FIG. 3 shows a sequence diagram for a method of measuring the charged amounts in a pressure container using pressure and volume according to an example for implementing the present invention.

THE BEST FORM FOR IMPLEMENTATION OF INVENTION

In the following, a desirable example for implementation of the present invention is described with reference to the attached drawings.

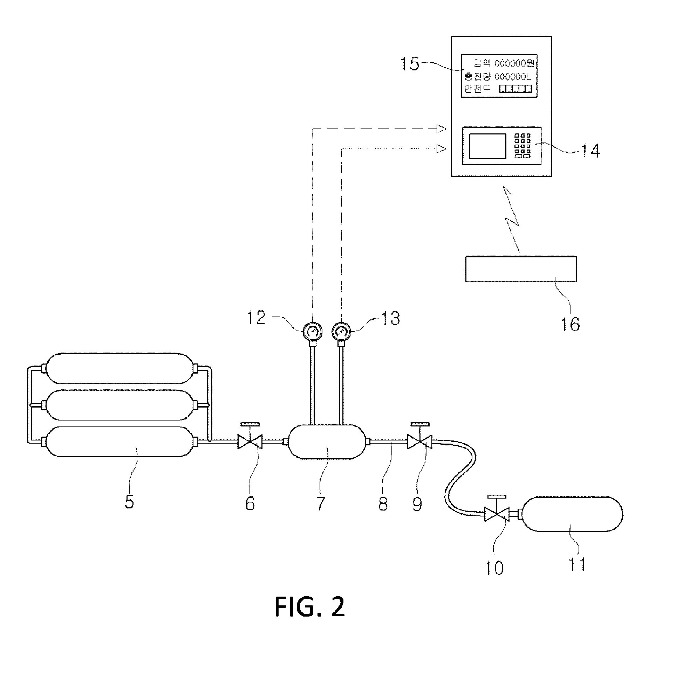

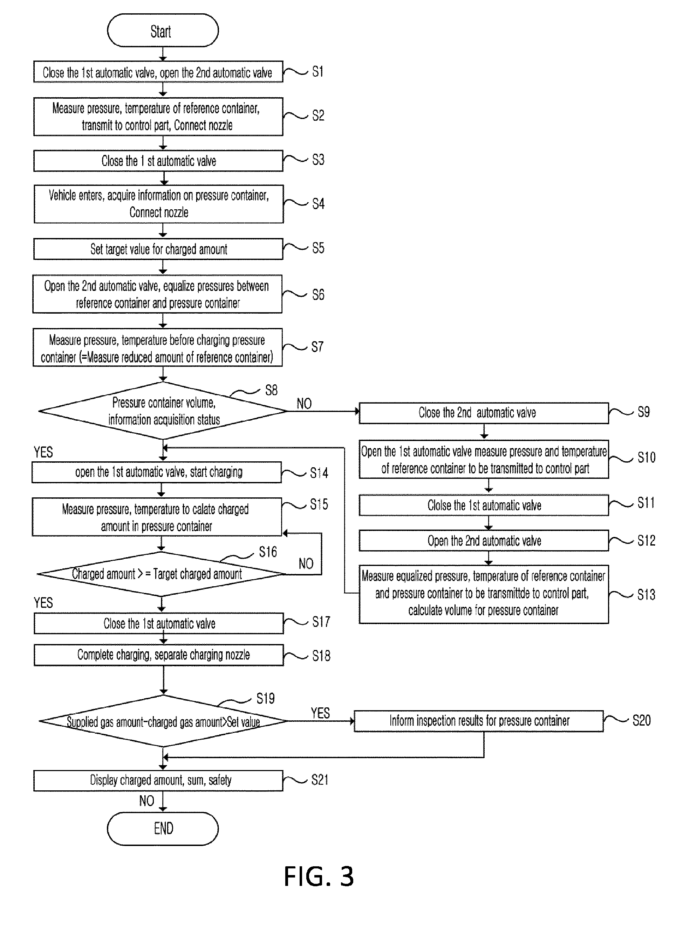

A measuring system and method for charged amounts in a pressure container using pressure and volume according to the present invention may be described as follows with reference to FIGS 2 and 3.

With reference to FIG. 2, the measuring system for charged amounts in the pressure container using pressure and volume according to the present invention is equipped with the first and second automatic valves (6,9), the reference container (7), pressure sensor (12), and temperature sensor (13) connected to the gas line (8) between the gas fuel storage tank (5) and the charging nozzle (10), and with the control part (14) that calculates a charged amount of gas fuel charged in the pressure container through engineering calculations with an input of volume information on the subject pressure container for charging gas fuel (11) along with a volume change of the pressure container to calibrate for the charged amount, determine safety of the pressure container and convert the charged amount to the sum, and include the display part (15) that informs a user of the information calculated and provided by the above control part as well as the information acquisition device of information on the pressure container that receives volume information of the pressure container (16).

The storage tank (5) is a tank that compresses gas fuels such as CNG or hydrogen gas (H2), etc. to a high pressure for storage, and generally corresponds to a gas storage place equipped in a gas charging station, while the pressure container (11) is installed at an automobile or a place that gas is used.

The first automatic valve (6) and second automatic valve (9) realize open/close motion by the control part (14), where the first automatic valve (6) is installed between the storage tank (5) and the reference container (7) to supply or shut off gas fuel of the storage tank to the reference container while the second automatic valve (9) is installed between the reference container (7) and the charging nozzle (10) to supply or shut off gas fuel of the reference container to the charging nozzle.

The reference container (7) is a configuration that is installed between the first automatic valve (6) and the second automatic valve (9) and provides a reference for detection of pressures and temperatures before and after charging the gas fuel in the pressure container (11). It is desirable to use a container with stability where a volume change resulting from changes in temperature or pressure is negligible.

The pressure sensor (12) and temperature sensor (13) are installed at the reference container (7) to detect pressure and temperature of the charged gas and transmit the pressure and the temperature to the control part (14).

The control part (14) calculates the charged amounts for gas supplied to a vehicle using equations according to the present invention based on the equation of state for an ideal gas with inputs of pressure and temperature detected by the pressure sensor (12) and the temperature sensor (13) or volume information of the pressure container (11) through ID card (RF card or barcode, etc.) or communication (charged vehicle and the present charger), and calculate volume changes in preparation for the past charged amount included in the history of pressure container (11) as well as pressure container volumes detected before and after charging gas fuel in the pressure container to determine safety based on the volume change while calibrating the above calculated charged amount. It is desirable to realize the control part (14) with a PCB consisting of a PLC (Programmable Logic Controller) or EPROM and Firmware.

The display part (15) conveys information provided on the charged amount of gas fuel calculated by the control part (14) along with the sum of money and safety through text or voice, where it is desirable to display the text via LCD while generating voice through a speaker.

As described above, the measuring system of charged amounts of a pressure container using pressure and volume per the present invention is characterized by charging for gas fuel having an arrangement of the reference container between the storage container and the charging nozzle and with operation of the first and second automatic valves.

Additionally, the system of the present invention for charging gas fuel in the pressure container without being equipped with a flow meter is characterized by affirmation of volume of the subject pressure container for charging and calculation of the amount of gas fuel remaining in the pressure container before charging, based on which the amount of gas fuel to be charged is verified and followed by charging.

To calculate the charged amount for gas fuel, volume information of the pressure container is first received using the information acquisition device (16) for the pressure container. The information acquisition device (16) is connected with the control part (14) and transmits the inputted volume information for the pressure container to the control part (14). This is because the volume information is required to calculate the charged amount based on the volume of the pressure container.

More specifically, information about volume, pressure and temperature of the pressure container is acquired by inputting from an ECU (vehicle), transmission through connection of an information communication cable with the charging nozzle as with hydrogen charging, or inputting from RF tag, bar code, etc. as with vehicles using a POS (Point of sale system). Such volume information of pressure containers may be used for evaluation of the charged amounts of fuel gas and the safety of pressure container.

With common pressure containers (11) for charging gas fuel, back draft preventing valves are ensured to be installed at an inlet for gas fuel to prevent leakage. Therefore, measurements of pressure and temperature of the gas fuel remaining in the pressure container (11) using the pressure sensor (12) and the temperature sensor (13) are impossible due to the pressure in the pressure container (11) even if the charging nozzle equipped in the charging system of the present invention is connected and the second automatic valve is opened.

Therefore when a separate reference container of pressure measurement is equipped to measure pressure and temperature of the gas fuel remaining in the pressure container (11) with the gas fuel charged in the reference container at a maximum possible pressure that may be prepared by the charging station and the charging nozzle connected, equal temperatures and equal pressures are formed by charging of gas of the reference container in the pressure container, and pressure and temperature of the reference container is measured to enable calculation of the amount of gas fuel remaining in the pressure container when temperatures and pressures of the reference container and the pressure container are identical.

In the following methods for charging gas fuel and measuring according to the present invention are described. A measuring method for the charged amounts in the pressure container using pressure and volume according to the present invention comprises detection steps (S1~S3) for initial pressure and temperature values of the reference container that closes the first automatic valve after gas fuel of the storage tank is supplied to the reference container by opening the first automatic valve with the second automatic valve closed and transmitted to the control part with detection of pressure and temperature of the reference container; a collection step for basic information (S4) where basic information is collected for the subject pressure container for supply of gas fuel; a setting step (S5) where a target value is set for charging the gas fuel in the pressure container; detection steps for pressure and temperature values (S6~S7) before charging the pressure container that equalizes pressures and temperatures for the reference container and the pressure container by opening the second automatic valve detects the pressure and the temperature under this condition and transmits them to the control part; a determination  step for charging implementation status (S8) to determine implementation status of the subsequent steps to find a starting to charge and the volume, depending on the acquisition status of volume information for the pressure container; acquisition steps for volume information of the pressure container (S9~S13) that calculate volume of the pressure container by measuring pressure and temperature of the reference container and transmitting them to the control part with closing of the second automatic valve and opening of the first automatic valve followed by the same with closing of the first automatic valve and opening of the second automatic valve to find volume of the pressure container, and measuring pressure and temperature with the pressure equalized for the reference container and the pressure container and transmitting them to the control part with closing of the first automatic valve and opening of the second automatic valve; a charging step to charge gas fuel in the pressure container from the storage tank (S14) by opening the first automatic valve after acquiring volume information of the pressure container; detection steps for pressure and temperature values (S15~17) after charging the pressure container where pressure and temperature are transmitted to the control part after their detection with the first automatic valve closed under a condition where the charged amount reaches the target charging value and gases of the pressure container and of the reference container are equalized in pressure and equal temperature; a charging completion step (S18) where the charging nozzle is separated from the pressure container; a calculation step of the charged amount for the pressure container (S 19) where the calibration amount of fuel gas is calculated according to the amount of gas fuel that moved from the reference container to the pressure container at the detection step of pressure and temperature before charging as well as a difference in gas fuel volumes before and after charging calculated by the control part to calculate the charged amount for the pressure container which is compared with the supplied amount to determine an excess status beyond the set value; and informing steps (S20~S21) where the pressure container condition is inspected with calculation for an agreement status between the charged amount and the set value, and the display part is informed of the sum converted from the charged amount as well as the safety through text or voice.

More specifically, a method of charging gas fuel in a vehicle using the measuring system, schematically shown in FIG. 2, for the charged amount of a pressure container using pressure and volume is described as an implementation example.

In the initial condition of the measuring system for the charged amount of a pressure  container using pressure and volume of the present invention, the first automatic valve (6) and the second automatic valve (9) are closed.

As shown in FIG. 3, the first automatic valve (6) is opened in the step for charging the high-pressure fuel gas in the reference container (S1). At this stage, gas in the storage tank (5) is moved to the reference container (7) for charging by the pressure difference between the high-pressure storage tank (5) and the reference container with a low pressure in the initial condition.

With pressure and temperature of the reference container (7) detected by the pressure sensor (12) and the temperature sensor (13), the initial pressure and temperature values are transmitted to the control part (14) (S2).

The first automatic valve (6) is closed (S3). The steps S1 through S3 correspond to preparation steps performed before charging the gas fuel.

It is followed by connecting the charging nozzle to the nozzle of the pressure container as a fuel tank for a vehicle when the vehicle enters a charging station (S4). A charged amount is then set (S5).

The second automatic valve (9) is opened and the first pressure equalization process is implemented between the said reference container (7) and the pressure container (11) (S6).

As the reference container (7) and pressure container (11) come to have equal pressures and temperatures, pressure and temperature are measured by the pressure sensor (12) and the temperature sensor (13), and the pressure and temperature values of the pressure container (11) before charging are transmitted to the control part (14) (S7).

Since there was movement of gas from the reference container (7) to the pressure container (11), the final amount charged of gas is compensated for the amount of moved gas. Namely, since the gas amount of the pressure container calculated in step S6 by the measured pressure and temperature values is an amount where the amount of moved gas from the reference container (7) to the pressure container (11) in step 6 is added. Thus, the actual charged amount in the pressure container (11) is calculated by adding the amount of moved gas to the pressure container (11) to the final charged amount. The steps S4 through S7 correspond to steps for measuring the pressure and temperature of the pressure container (11) before charging.

It is followed by acquiring volume information of the pressure container (11) from ECU, RF tag, bar code, etc. through an information acquisition device, after which charging in a high-pressure container is started. When volume information of the pressure container is not known, the volume is calculated by steps S9~S13 (S8). At this time, basic information about the pressure container acquired by the information acquisition device may include volume information of the pressure container, information on a maximum charging pressure, current pressure and temperature, etc. for instance.

When the reference container (7) and pressure container (11) come to have an equal pressure, temperature and pressure in the reference container (7) and the pressure container (11) become equal. Since pressure, volume and temperature of the reference container (7) are first known, volume of the pressure container (11) may be derived by the equation 13 as explained later.

It is followed by closing of the second automatic valve (9) (S9).

And the first automatic valve (6) is opened to supply gas fuel from the storage tank (5) to the reference container (7) and pressure along with temperature of the reference container (7) is measured followed by transmission to the control part (S10) and closing of the first automatic valve (6) (S11).

When the second automatic valve (9) is opened and gas fuel is moved from the reference container (7) to the pressure container (11), the second pressure equalization process for the reference container (7) and the pressure container (11) proceeds once more (S13).

When the reference container (7) and the pressure container (11) come to have equal pressures as a result of the second pressure equalization process, temperature and pressure in the reference container (7) and the pressure container (11) become identical. Since pressure, volume and temperature of the reference container (7) are first known, gas volume of the pressure container (11) may be derived also by the equation 13 as described later. Steps S9 through S13 correspond to the steps for measuring the volume of the pressure container (11).

When volume of the pressure container (11) is acquired by ECU, RF tag, bar code or the processes S9~S13, the first automatic valve (6) is opened and charging is started (S14).

To calculate the charged amounts for the pressure container with the pressure sensor (12) and the temperature sensor (13), values are continuously measured (S15). Through engineering calculations, these values are compared with the target value for charged amount to determine the conclusion point of charging (S16).

Upon completion of charging, the first automatic valve is closed (S 17). The charging nozzle (10) is separated from the pressure container (S18).

Also, the charging information for each vehicle is stored and since leakage of the vehicle or the pressure container (11) or permanent deformation of the pressure container may be suspected when the target value upon charging exceeds the charged amount per specification, legal pressure, or the charged amount in the past, charging is suspended and the user is informed through an alarm so that follow-up actions may be devised (S20). For instance, safety of the pressure container (11) may be indicated on the display part (15) with phrases such as safe, dangerous, limit value approached, etc., or safe, dangerous, limit value approached, etc. is output by voice through a speaker(not shown) included in the display part (15), or by measurements of the extent of safety with a graduated gauge.

And the control part (14) is separately equipped with the display part (15) to indicate the charged amount of gas and the converted sum of money (S21).

Therefore, the present invention has the effect of resolving problems of long time requirements for conventional inspection of natural gas containers so that safety of the pressure container may be verified within about 1-3 minutes required upon gas charging, and of enabling routine checkups rather the legally stipulated periodic checkups as well as preventing explosion accidents in advance by prevention of charging in a container of questionable safety.

The equation for calculation of a volume of the gas charged in the pressure container (11) is as follows.

In the present invention, when the reference container (7) and the pressure container have equal pressures, volume of the pressure container (11) may be obtained by the equation 13, since the pressure and the temperature are identical and volume of the reference container (7) is known. When the actual condition is converted to the standard state in the equation of state for an ideal gas to obtain gas volume for the reference container (7) and the pressure container (11), the gas amount charged in the pressure container (11) may be obtained.

Standard state refers to a condition at 0° C, 1 atm, and volume of gas molecules is determined by the number of mols. Hereafter, the subscript 1 refers to the standard state while the subscript 2 denotes the actual state. Pressure and temperature as an actual state of the reference container (7) are measured by the pressure sensor (12) and the temperature sensor (13).

P2=PKPa
V2 = Actual Volume Liter

$$T2 = T \; K \; (T \; ^oC + 273.15 \; K)$$

Equations of the reference container (7) in the standard state and the actual state are as shown by Equations 1 and 2, respectively. In the following equations 1 and 2, the subscript denotes the reference container (7).

$$P_{1c}V_{1c} = nRT_{1c} \quad \cdots\cdots\cdots \qquad \text{(Equation 1)}$$

$$P_{2c}V_{2c} = nRT_{2c} \quad \cdots\cdots\cdots \qquad \text{(Equation 2)}$$

Calculation of the volume in the standard state as the volume at the actual state is shown by Equation 3.

$$\frac{P_{1c}V_{1c}}{T_{1c}} = nR \quad , \quad \frac{P_{2c}V_{2c}}{T_{2c}} = nR$$

$$\frac{P_{1c}V_{1c}}{T_{1c}} = \frac{P_{2c}V_{2c}}{T_{2c}} = nR$$

$$V_{1c} = \frac{P_{2c}V_{2c}}{T_{2c}} \times \frac{T_{1c}}{P_{1c}} = V_{2c} \times \frac{P_{2c}}{P_{1c}} \times \frac{T_{1c}}{T_{2c}} \quad \cdots\cdots\cdots \qquad \text{(Equation 3)}$$

Similarly, for the pressure container (12), calculation of the volume in the standard state is shown by Equation 4. In Equation 4 below, the subscript denotes the pressure container (11).

$$P_{1v}V_{1v} = nRT_{1v} , \quad P_{2v}V_{2v} = nRT_{2v}$$

$$\frac{P_{1v}V_{1v}}{T_{1v}} = nR \quad , \quad \frac{P_{2v}V_{2v}}{T_{2v}} = nR$$

$$\frac{P_{1v}V_{1v}}{T_{1v}} = \frac{P_{2v}V_{2v}}{T_{2v}} = nR$$

$$V_{1v} = \frac{P_{2v}V_{2v}}{T_{2v}} \times \frac{T_{1v}}{P_{1v}} = V_{2v} \times \frac{P_{2v}}{P_{1v}} \times \frac{T_{1v}}{T_{2v}} \quad \cdots\cdots\cdots \qquad \text{(Equation 4)}$$

Calculation of the volume in the standard state under the condition where equal pressures are realized is as shown by Equation 5. In Equation 5, the subscript indicates the condition where the reference container (7) and the pressure container (11) have equal pressures.

$$P_{1t}V_{1t} = nRT_{1t} \, , \;\; P_{2t}V_{2t} = nRT_{2t}$$

$$\frac{P_{1t}V_{1t}}{T_{1t}} = nR \;\; , \;\; \frac{P_{2t}V_{2t}}{T_{2t}} = nR$$

$$\frac{P_{1t}V_{1t}}{T_{1t}} = \frac{P_{2t}V_{2t}}{T_{2t}} = nR$$

$$V_{1t} = \frac{P_{2t}V_{2t}}{T_{2t}} \times \frac{T_{1t}}{P_{1t}} = V_{2t} \times \frac{P_{2t}}{P_{1t}} \times \frac{T_{1t}}{T_{2t}} \qquad \cdots \cdots \cdots \qquad \text{(Equation 5)}$$

Since, according to the mass-energy preservation law, the sum of volumes in the standard state is identical under the condition where equal pressures are realized, the following relationships are obtained.

$$V_{1t} = V_{1c} + V_{1v} \qquad \cdots \cdots \cdots \qquad \text{(Equation 6)}$$

$$V_{2t} = V_{2c} + V_{2v} \qquad \cdots \cdots \cdots \qquad \text{(Equation 7)}$$

Substituting Equation 7 in Equation 5, Equation 8 is obtained.

$$V_{1t} = V_{2c} \times \frac{P_{2c}}{P_{1c}} \times \frac{T_{1c}}{T_{2c}} + V_{2v} \times \frac{P_{2v}}{P_{1v}} \times \frac{T_{1v}}{T_{2v}} = V_{2t} \times \frac{P_{2t}}{P_{1t}} \times \frac{T_{1t}}{T_{2t}} \qquad \text{(Equation 8)}$$

$$V_{1t} = V_{2c} \times \frac{P_{2c}}{P_{1c}} \times \frac{T_{1c}}{T_{2c}} + V_{2v} \times \frac{P_{2v}}{P_{1v}} \times \frac{T_{1v}}{T_{2v}} = (V_{2c} + V_{2v}) \times \frac{P_{2t}}{P_{1t}} \times \frac{T_{1t}}{T_{2t}} \qquad \text{(Equation 9)}$$

By putting

$$\frac{T_{1c}}{T_{2c}} = A \, , \; \frac{T_{1v}}{T_{2v}} = B, \; \frac{T_{1t}}{T_{2t}} = C \, ,$$

then

$$V_{1t} = V_{2c} \times \frac{P_{2c}}{P_{1c}} \times A + V_{2v} \times \frac{P_{2v}}{P_{1v}} \times B = (V_{2c} + V_{2v}) \times \frac{P_{2t}}{P_{1t}} \times C$$

$$V_{2c} \times \frac{P_{2c}}{P_{1c}} \times A + V_{2v} \times \frac{P_{2v}}{P_{1v}} \times B = V_{2c} \times \frac{P_{2t}}{P_{1t}} \times C + V_{2v} \times \frac{P_{2t}}{P_{1t}} \times C$$

$$V_{2v} \times \frac{P_{2v}}{P_{1v}} \times B - V_{2v} \times \frac{P_{2t}}{P_{1t}} \times C = V_{2c} \times \frac{P_{2t}}{P_{1t}} \times C - V_{2c} \times \frac{P_{2c}}{P_{1c}} \times A$$

$$V_{2v} \times \left( \frac{P_{2v}}{P_{1v}} \times B - \frac{P_{2t}}{P_{1t}} \times C \right) = V_{2c} \times \left( \frac{P_{2t}}{P_{1t}} \times C - \frac{P_{2c}}{P_{1c}} \times A \right) \quad \cdots \qquad \text{(Equation 10)}$$

Since the pressure in the standard state is 1 atm, substitution in Equation 10 gives Equation 11.

$$P_{1v} = P_{1t} = P_{1c} = 1 atm = 101.325 \, KPa$$

$$V_{2v} \times \left( \frac{P_{2v} \times B - P_{2t} \times C}{P_{1v}} \right) = V_{2c} \times \left( \frac{P_{2t} \times C - P_{2c} \times A}{P_{1v}} \right) \quad \cdots \qquad \text{(Equation 11)}$$

$$V_{2v} = V_{2c} \times \left( \frac{P_{2t} \times C - P_{2c} \times A}{P_{1v}} \right) \times \left( \frac{P_{1v}}{P_{2v} \times B - P_{2t} \times C} \right)$$

Thus the volume in the actual state is given by Equation 12.

$$V_{2v} = V_{2c} \times \left( \frac{P_{2t} \times C - P_{2c} \times A}{P_{2v} \times B - P_{2t} \times C} \right) \quad \cdots \qquad \text{(Equation 12)}$$

Assuming that an equilibrium was achieved at

$$T_{1c} = T_{1v} = T_{1t} = 273.15 K$$

$$T_{2c} = T_{2v} = T_{2t} = 293.15 K$$

Equation 13 is obtained since A=B=C where

$$V_{2v} = V_{2c} \times \left( \frac{A \times (P_{2t} - P_{2c})}{A \times (P_{2v} - P_{2t})} \right)$$

$$V_{2v} = V_{2c} \times \left( \frac{P_{2t} - P_{2c}}{P_{2v} - P_{2t}} \right) \quad \cdots \quad \text{(Equation 13)}$$

For instance, the reference container (7) is charged at 25,000 KPa, 5 liters, while the initial pressure and temperature of the pressure container (11) are unknown. When the reference container (7) and the pressure container (11) come to have equal pressures, the measured pressure is 6134.75 KPa and the temperature 2 (S7 step). As the second automatic valve (9) is closed and the first automatic valve (6) opened, the pressure of 25,000 KPa is charged in the reference container (S9, S10 steps). The first automatic valve (6) is closed and the second automatic valve opened to achieve equal pressures to the pressure container (11) (S11, S12 steps). The pressure upon achieving equal pressures is 6268.55 KPa and the temperature 20 °C (S13 step). Using the above information, substituting the volume of the pressure container (11) in Equation 13 gives the following result.

By Equation 13, the actual volume of the pressure container (11) is given as

$$V_{2v} = V_{2c} \times \left( \frac{P_{2t} - P_{2c}}{P_{2v} - P_{2t}} \right) \quad \cdots \quad \text{(Equation 13)}$$

$$V_{2v} = 5\,l \times \left( \frac{(6,268.55 + 101.325)\,KPa - (25,000 + 101.325)\,KPa}{(6,134.75 + 101.325)\,KPa - (6,268.55 + 101.325)\,KPa} \right)$$

$$V_{2v} = 5\,l \times \left( \frac{6,369.87\,KPa - 25,101.325\,KPa}{6,236.07\,KPa - 6,369.87\,KPa} \right)$$

$$V_{2v} = 700\,l$$

Therefore, the actual volume of the pressure container may be calculated to be 700 liters. When volume of the reference container (7), initial pressure and temperature are known and a vehicle is stocked in a gas charging station, volume of the gas stored in the pressure container (11) of the charged vehicle may be obtained by measuring initial pressure and temperature as the first equal pressure, and by measuring pressure and temperature after achieving the second equal pressure in the reference container (7).

The charged amount may be calculated using the volume and pressure of the container when the vehicle was initially stocked and the difference in pressure after completion of charging.

A vehicle without knowledge of volume, initial temperature and pressure of the pressure container (11) has been stocked. In the S6 step, equal pressures to the reference container (7) have been realized, giving 6,134.75 kPa as measured by the pressure sensor (12) and 20 °C by the temperature sensor (13), and thus the state of the pressure container (11) before charging may be seen.

The actual volume of the pressure container (11) has been obtained by Equation 13 to be 700 liters. Or, volume information may also be acquired by ID card, etc. The volume in the standard state before charging is as follows. In the Equation below, the subscript "i" denotes an initial state.

In Equation 4,

$$V_{1v} = \frac{P_{2v} V_{2v}}{T_{2v}} \times \frac{T_{1v}}{P_{1v}} = V_{2v} \times \frac{P_{2v}}{P_{1v}} \times \frac{T_{1v}}{T_{2v}} \quad \cdots \cdots \quad \text{(Equation 4)}$$

$$V_{1i} = 700\,l \times \frac{6134.75\,KPa + 101.325\,KPa}{101.325\,KPa} \times \frac{273.15\,K}{20\,℃ + 273.15\,K}$$

$$V_{1i} = 700\,l \times \frac{6236.075\,KPa}{101.325\,KPa} \times \frac{273.15\,K}{293.15\,K}$$

$$V_{1i} = 40,142.48\,l$$

If buffered with 20,000 KPa (g), temperature of 20 °C, volume in the standard state upon being buffered by Equation 4 is as follows.

In the Equation below, the subscript "r" denotes the state of completed charging.

$$V_{1v} = \frac{P_{2v}V_{2v}}{T_{2v}} \times \frac{T_{1v}}{P_{1v}} = V_{2v} \times \frac{P_{2v}}{P_{1v}} \times \frac{T_{1v}}{T_{2v}} \qquad \cdots\cdots\cdots \quad \text{(Equation 4)}$$

$$V_{1f} = 700\,l \times \frac{20,000\,KPa + 101.325\,KPa}{101.325\,KPa} \times \frac{273.15\,K}{20°C + 273.15\,K}$$

$$V_{1f} = 700\,l \times \frac{20,101.325\,KPa}{101.325\,KPa} \times \frac{273.15\,K}{293.15\,K}$$

$$V_{1f} = 129,394.98\,l$$

The charged amount from the reference container (7) to the pressure container (11) in the S6 step process to measure the initial pressure and temperature of the pressure container (11) is as follows.

Since the reference container (7) was initially (S2 step) at 25,000 kPa, 5 liters, 20 °C and the pressure container (11) realized an equal pressure at 6,134.75 KPa (g) (S5 step), the pressure difference is 18,865.24 KPa.

$$P = 25,000\ KPa - 6,134.75\ KPa = 18,865.24\ KPa$$

The subscript "cf" indicates a reduced amount of the reference container(7), i.e., the charged amount to measure the initial pressure of the pressure container (11).

In Equation 4,

$$V_{1cf} = 5\,l \times \frac{18,865.24\,KPa + 101.325\,KPa}{101.325\,KPa} \times \frac{273.15\,K}{20°C + 273.15\,K}$$

$$V_{1cf} = 5\,l \times \frac{18966.57\,KPa}{101.325\,KPa} \times \frac{273.15\,K}{273.15\,K}$$

$$V_{1cf} = 872.07\,l$$

Therefore, for total charged amount, as much as the difference between the final calculated volume in the standard state and the volume in the initial standard state has been charged, and it was also charged more by the reduced amount of the reference container supplied to measure initial temperature and pressure.

$$Vs = V_{1f} - V_{1i} + V_{cf} = 129,394.98\,l - 40,142.48\,l + 872.07\,l = 90124.57\,l$$

In summary of the above process, a vehicle without knowledge of volume, initial temperature, pressure of the pressure container (11) is stocked (S4 step). For the reference container (7), 5 liters along with the initial pressure of 25,000 KPa are known (S2 step). Upon pressure equalization to the reference container (7) the volume is found to be 700 liters by Equation 13 using the initial temperature of 20 °C, pressure of 6,134.75 KPa (S5, S6, S7 steps). Afterwards, it is charged at 20 °C to the pressure of 20,000 KPa. Namely, the pressure container is charged from 6,134.752 KPa up to 20,000 KPa. Hence, a total charged amount may be found by calculating the changed amount in volume in the standard state according to the pressure change, i.e., the charged amount and adding to this the charged amount to achieve pressure equalization in the reference container (7) for measurement of the initial pressure.

As shown above, the initial pressure, temperature, actual volume of the stocked pressure container (11) may be obtained using the reference container (7) even if volume of the pressure container is not known, and the charged amount may be calculated by measuring pressure, temperature before/after charging without a mass flow meter.

Also, the present invention applies the compensation range according to temperature changes to precisely calculate the charged amount of gas.

Assuming 700 liters, 20,000 KPa, 0 °C for the pressure container (11), the standard volume is obtained as follows when there is no temperature compensation.

$$V_1 = 700\,l \times \frac{20,000\,KPa + 101.325\,KPa}{101.325\,KPa} \times \frac{273.15\,K}{0\,°C + 273.15\,K}$$

$$V_1 = 700\,l \times \frac{20,101.325\,KPa}{101.325\,KPa} \times \frac{273.15\,K}{273.15\,K}$$

$$V_1 = 138,869.26\,l$$

The subscript "co" represents a state with temperature compensation. When the pressure container (11) is assumed to be charged to 700 liters, 20,000 KPa, and 20 °C, the standard volume is obtained as follows.

$$V_{1co} = 700\,l \times \frac{20,000\,KPa + 101.325\,KPa}{101.325\,KPa} \times \frac{273.15\,K}{20\,°C + 273.15\,K}$$

$$V_{1co} = 700\,l \times \frac{20,101.325\,KPa}{101.325\,KPa} \times \frac{273.15\,K}{293.15\,K}$$

$$V_{1co} = 129,394.95\,l$$

Similarly, when hydrogen is charged in the container of 700 liters to 70,000 KPa, summarizing the cases of compensating temperatures of 0 °C and 20 °C gives rise to the Table 1 below.

[Table 1]

| Case | Charging Pressure (KPa) | Temperature (°C) | Pressure Container (L) | Volume in Standard State (L) |
|------|--------------------------|-------------------|-------------------------|-------------------------------|
| A(CNG) | 20,000 | 0 | 700 | 138,869.28 |
| B(CNG) | 20,000 | 20 | 700 | 129,394.95 |
| C(H2) | 70,000 | 0 | 700 | 484,292.40 |
| D(H2) | 70,000 | 20 | 700 | 451,251.81 |

Therefore, Vd or the difference in standard volumes with and without consideration of the temperature of 20 °C when a vehicle with a pressure container of 700 liters, is charged to the state of 20,000 KPa is

$$V_d = V_1 - V_{co} = 138,869.28\,l - 129,394.95\,l = 9474.33\,l$$

If the difference upon compensating for temperatures of 0 °C and 20 °C is calculated when a high pressure of 70,000 KPa is charged in the pressure container of 700 liters,

$$V_d = V_1 - V_{co} = 484,292.40\,l - 451,251.81\,l = 33040.59\,l$$

Thus, since the error increases as the charging pressure increases, the charged gas amount should be accurately compensated considering temperature. To compensate the calculated charged amount of gas, temperature for compensation is obtained by the temperature sensor (13), and inputted in the control part (14). Since the unit price of gas is per $m^3$ and price differences of 9.47 $m^3$ in the case of CNG and of 33.04 $m^3$ in the case of hydrogen occur, temperatures is ensured to be always compensated for.

For conversion of the charged amounts according to temperature changes, volume changes in the standard state with temperatures is calculated beforehand as illustrated in Table 1 for comparative determination by the control part (14). Since data on the changed amounts according to the relevant temperature are stored in the control part (14) in advance, the amount of volume to be compensated for may be calculated as shown in Table 1, by the control part (14) recalling the volume change data according to temperatures from the already stored volume change data as a function of temperature difference when temperatures are read by the temperature sensor (13).

Also, the compensation method according to volume changes of the pressure container (11) is calculated as follows.

High-pressure containers used as a pressure container for vehicles may be largely classified into four types, with descriptions of characteristics for each shown in Table 2.

Table 2

| Kind | Characteristics | Remarks |
| --- | --- | --- |
| Type 1 (Metals) | Heavy, internal corrosion | Produced only in metals |
| Type 2 (Metals+ Reinforced with rear wrap) | Heavy, prevention of internal corrosion required | Metal liner with only center body part of container reinforced by glass fiber composite materials |
| Type 3 (Metals +Reinforced with full wrap) | Light, prevention of galvanic corrosion required | Seamless thin aluminum liner with entire container reinforced with carbon fiber composite materials |
| Type 4 (Nonmetals + Reinforced with full wrap) | Lightest, excellent safety, longest use life | Plastic liner with entire container reinforced with carbon fiber composite materials |

For pressure containers for vehicles (11), types 3 and 4 have minute deformation while types 1 and 2 have almost no deformation. Amount of such deformation is determined by the maximum allowed stress, and there may be no large deformation due to the characteristics of the pressure container (11). Hence, based on the container of 700 liters at 20,000 KPa, 20 °C, compensation is to be made with conversion of the charged amount under the assumption of changes in volume by 0.1%, 0.5%, 1%, respectively, upon charging.

When charging is completed for the pressure container of 700 liters, 20,000 KPa, 20 °C, conversion to the standard volume gives the following results. The subscript "f" indicates the state of completed charging.

$$V_{1f} = 700\,l \times \frac{20,000\,KPa + 101.325\,KPa}{101.325\,KPa} \times \frac{273.15\,K}{20°C + 273.15\,K}$$

$$V_{1f} = 700\,l \times \frac{20,101.325\,KPa}{101.325\,KPa} \times \frac{273.15\,K}{293.15\,K}$$

$$V_{1f} = 129{,}394.98\,l$$

When the volume was deformed by 0.01 % (0.07 liters)

$$V_{1f} = (700\,l + 0.07\,l) \times \frac{20{,}000\,KPa + 101.325\,KPa}{101.325\,KPa} \times \frac{273.15\,K}{20\,^{\circ}\!C + 273.15\,K}$$

$$V_{1f} = 700.07\,l \times \frac{20{,}101.325\,KPa}{101.325\,KPa} \times \frac{273.15\,K}{293.15\,K}$$

$$V_{1f} = 129{,}407.92\,l$$

Similarly, considering deformations by 0.05%, 0.1%, the changes in standard volume according to volume changes of the pressure container (11) are as shown in Table 3.

Table 3

| Case | A | B | C | D |
|---|---|---|---|---|
| Amount of deformation (%) | 0% | 0.01% | 0.05% | 0.1% |
| Container volume (liter) | 700 | 700.07 | 700.35 | 700.7 |
| Standard volume(liter) | 129,394.98 | 129,407.92 | 129,459.67 | 129,524.37 |
| Difference from case A (liter) | 0 | 12.94 | 64.69 | 129.39 |

In the above Table 3, the amount of deformation as a function of volume changes of the container has a very small value in comparison with the standard volume.

In the case of D, when the pressure container was deformed in volume by 0.1% and charged to the maximum charging pressure of 20,000 KPa, an additional 0.13 m$^3$ was supplied in preparation for the container volume without deformation.

Namely, even if the pressure container (11) is charged to a maximum charging pressure, there is no large difference in sales volume in terms of conversion dependent on the volume. Each container manufacturer is inspected and formally approved by national institutions concerning the pressure containers. At this time, material properties and deformation characteristics are supposed to be submitted, and this information is collected by the control part (14) and utilized as the basic data to affirm deformation and safety of the pressure container (11). The control part (14) stores material properties and deformation characteristic values per pressure container in advance, and may select the characteristic values of the relevant pressure container upon calculation of charged amounts depending on the information of the pressure container read off of the information acquisition device (16) for the pressure container or may read off of RF tag of the vehicle in the information acquisition device (16) of the pressure container (when information on material properties and deformation characteristics is stored in the RF tag).

For conversion of the charged amounts according to the volume increase, the changes in standard volume as a function of the container volumes are calculated beforehand and comparatively determined by the control part (14) as illustrated in Table 3.

Therefore, the present invention may accurately calculate the charged amount of gas by compensating for errors in gas volume of the pressure container (11) according to temperature changes or volume changes of the pressure container using the pressure sensor and pre-registered information of the container.

Determination of safety of the pressure container (11) is performed under the premise that volume of the pressure container is known. For instance, the pressure container (11) has the volume of 700 liters at 1,000 KPa, 20 °C while the reference container(7) is 5 liters in volume at 25,000 KPa, 20 °C. According to Equation 4 described earlier, standard state volume of the pressure container (11) is

$$V_{1v} = \frac{P_{2v} V_{2v}}{T_{2v}} \times \frac{T_{1v}}{P_{1v}} = V_{2v} \times \frac{P_{2v}}{P_{1v}} \times \frac{T_{1v}}{T_{2v}} \qquad \cdots \cdots \cdots \qquad \text{(Equation 4)}$$

$$V_{1v} = 700\,l \times \frac{1{,}000\,KPa + 101.325\,KPa}{101.325\,KPa} \times \frac{273.15\,K}{20\,℃ + 273.15\,K}$$

$$V_{1v} = 700\,l \times \frac{1{,}101.325\,KPa}{101.325\,KPa} \times \frac{273.15\,K}{293.15\,K}$$

$$V_{1v} = 65{,}023.61\,l$$

According to Equation 3, the volume of the reference container (7) in standard state is given by

$$V_{1c} = \frac{P_{2c} V_{2c}}{T_{2c}} \times \frac{T_{1c}}{P_{1c}} = V_{2c} \times \frac{P_{2c}}{P_{1c}} \times \frac{T_{1c}}{T_{2c}} \qquad \cdots \cdots \cdots \qquad \text{(Equation 3)}$$

$$V_{1c} = 5\,l \times \frac{25{,}000\,KPa + 101.325\,KPa}{101.325\,KPa} \times \frac{273.15\,K}{20\,℃ + 273.15\,K}$$

$$V_{1c} = 5\,l \times \frac{25{,}101.325\,KPa}{101.325\,KPa} \times \frac{273.15\,K}{293.15\,K}$$

$$V_{1c} = 1{,}154.15\,l$$

Standard volume upon pressure equalization is given by Equation 6.

$$V_{1t} = V_{1c} + V_{1v} \qquad \cdots \cdots \cdots \qquad \text{(Equation 6)}$$

$$V_{1t} = 65{,}023.61\,l + 1{,}154.15\,l = 66{,}177.76$$

Upon achievement of the equalized pressure, the actual volume is calculated by Equation 7.

$$V_{2t} = V_{2c} + V_{2v} \qquad \cdots \cdots \cdots \qquad \text{(Equation 7)}$$

$$V_{2t} = V_{2c} + V_{2v} = 5\,l + 700\,l = 705\,l$$

By arranging Equation 5 for pressure upon achievement of the equalized pressure,

$$V_{1t} = \frac{P_{2t} V_{2t}}{T_{2t}} \times \frac{T_{1t}}{P_{1t}} = V_{2t} \times \frac{P_{2t}}{P_{1t}} \times \frac{T_{1t}}{T_{2t}} \qquad \cdots \cdots \cdots \qquad \text{(Equation 5)}$$

$$P_{2t} = P_{1t} \times \frac{V_{1t}}{V_{2t}} \times \frac{T_{2t}}{P_{1t}} \quad \cdots\cdots\cdots \qquad \text{(Equation 14)}$$

$$P_{2t} = 101.325\,KPa \times \frac{66{,}177.76\,l}{705\,l} \times \frac{293.15}{273.15} = 10{,}207.70\,KPa$$

Hence, when volume information of the pressure container (11) is acquired, pressure and temperature are known by measurements, and volume and temperature of the reference container are known, the equalization pressure may be determined by engineering calculations. The value of 10,207.70 KPa obtained by Equation 14 is a theoretical pressure value. If the value measured by the pressure sensor (12) upon pressure equalization is compared with the calculated theoretical value, then safety of the pressure container may be determined. If the theoretical pressure value is lower than the pressure value calculated by measurement, it corresponds to either the condition of an increased container size or a case of gas leakage. On the other hand, when the theoretical pressure is higher than the set value, it represents a case of a reduced volume for the pressure container, etc., which enables determination of a deformation status of the pressure container.

As above, if volume information of the pressure container (11) is known, then safety of the pressure container may be verified by the reference container (7) and the measuring method of the present invention for charged amounts in the pressure container using pressure and volume.

Those with ordinary skill in the technology area to which the present invention belongs will understand that the above descriptions are illustrative in all aspects without limitations. The scope of the present invention is revealed by the patent claims described later rather than by the description in detail. Particularly, the meaning and the scope of the claims and all changes or all transformed forms derived from the equivalents are included in the scope of the present invention.

**Claims**

1. A measuring system for charged amounts in a pressure container using pressure and volume, comprising:

    an information acquisition device for the pressure container that receives volume and temperature information of the pressure container,
    a first and second automatic valves, a reference container, a pressure sensor and a temperature sensor connected to a gas line between a gas storage tank and a charging nozzle,
    a control part that receives pressure detected by the pressure sensor, temperature detected by the temperature sensor and basic information of the pressure container for gas charging to calculate the charged amounts of gas charged in the pressure container and changed amounts in temperature and volume of the pressure container to calibrate for the charged amounts, to determine safety of the pressure container, and to convert the calibrated charged amount to a sum of money, and
    a display part that is supplied with the information calculated by the control part and informs a user of the system for measuring charged amounts in the pressure container.

2. The measuring system for charged amounts in a pressure container using pressure and volume of claim 1, **characterized by**
    the information acquisition device connected to the control part, the information acquisition device receiving volume information of the pressure container and transmitting it to the control part,
    the first automatic valve being installed between the storage tank and the reference container and supplying or shutting off gas in the storage tank to the reference container,
    the second automatic valve being installed between the reference container and the charging nozzle and supplying or shutting off gas in the reference container to the charging nozzle,
    the reference container being installed between the first automatic valve and the second automatic valve and providing a reference for detecting pressure and temperature before and after charging gas fuel in the pressure container,
    the pressure sensor and the temperature sensor being installed in the reference container and detecting pressure and temperature, respectively, of the charged gas fuel,
    the control part being connected with the pressure sensor and the temperature sensor and calculating safety, charged amount and charged sum to provide them to the display part, and
the display part being connected with the control part and conveying information provided by the control part through text or voice.

3. A measuring method for charged amounts of fuel gas in a pressure container, the measuring method for charged amounts of fuel gas in a pressure container using pressure and volume, **characterized by** measuring pressure and temperature of charged fuel gas without using a mass flow meter but using a reference container, and calculating the charged amounts by receiving an input with a volume of the pressure container or by measurement.

4. The measuring method for charged amounts in a pressure container using pressure and volume of claim 3, further comprising:

detection steps for initial pressure and temperature values of the reference container where gas fuel of a storage tank is supplied to the reference container by opening a first automatic valve with a second automatic valve closed, pressure and temperature of the reference container are detected and transmitted to the control part, followed by closure of the first automatic valve;
a collection step of basic information where basic information of the pressure container supplying gas fuel is collected;
a setting step where a target charging value is set for gas fuel charged in the pressure container;
detection steps for pressure and temperature values before charging the pressure container where pressure and temperature of the reference container and the pressure container are equalized by opening the second automatic valve, and the pressure and the temperature in this condition are detected and transmitted to the control part;
a determination step for determining whether to implement subsequent steps to find a case for starting to charge and the volume depending on an information acquisition status of the pressure container;
volume information acquisition steps for the pressure container where pressure and temperature of the reference container are measured and transmitted to the control part with the second automatic valve closed and the first automatic valve opened to find a volume of the pressure container, and where pressure and temperature are measured and transmitted to the control part with the first automatic valve closed and the second automatic valve opened to equalize pressures of the reference container and the pressure container so that the volume of the pressure container may be calculated;
a charging step of the pressure container where gas fuel is charged from the storage tank to the pressure container by opening the first automatic valve after acquiring volume information of the pressure container;
detection steps for pressure and temperature values after charging the pressure container where pressure and temperature are transmitted to the control part after their detection with the first automatic valve closed under a condition where the charged amount reaches the target charging value and gases of the pressure container and of the reference container are equalized in pressure and equal temperature;
a charging completion step where the charging nozzle is separated from the pressure container;
a calculation step for the charged amount in the pressure container where, in addition to the calculated difference in gas fuel volumes before and after charging calculated in the control part, a calibration amount for fuel gas according to an amount of gas fuel moved from the reference container to the pressure container at the detection step for pressure and temperature before charging as well as the changes in temperature or volume of the pressure container is calculated for calculation of the charged amount in the pressure chamber, which is compared with the supplied amount to determine whether the charged amount exceeds a set value; and
informing steps where agreement status between the charged amount and the set value for the pressure container is calculated for inspection of the pressure container conditions, and a sum of money converted from the charged amount and the safety are conveyed by a display part through text or voice.

5. The measuring method for charged amounts in a pressure container using pressure and volume of claim 4, further comprising:

an inputting step of the volume information and maximum charging pressure information for the pressure container by the control part using an ECU, RF tag or POS in a collection step of basic information, and
a step where safety abnormality for the pressure container is determined and such information is transmitted to the display part to generate alarm sounds when a difference between an amount of gas supplied by the reference container and the amount of gas fuel supplied to the pressure container exceeds the set value in the calculation step for the charged amount in the pressure container.

Prior Art

FIG. 1

FIG. 2

Start

Close the 1st automatic valve, open the 2nd automatic valve — S1

Measure pressure, temperature of reference container, transmit to control part, Connect nozzle — S2

Close the 1 st automatic valve — S3

Vehicle enters, acquire information on pressure container, Connect nozzle — S4

Set target value for charged amount — S5

Open the 2nd automatic valve, equalize pressures between reference container and pressure container — S6

Measure pressure, temperature before charging pressure container (=Measure reduced amount of reference container) — S7

Pressure container volume, information acquisition status — S8

NO → Close the 2nd automatic valve — S9

Open the 1st automatic valve measure pressure and temperature of reference container to be transmitted to control part — S10

Cloise the 1st automatic valve — S11

Open the 2nd automatic valve — S12

Measure equalized pressure, temperature of reference container and pressure container to be transmittde to control part, calculate volume for pressure container — S13

YES

open the 1st automatic valve, start charging — S14

Measure pressure, temperature to calate charged amount in pressure container — S15

Charged amount > = Target charged amount — S16    NO

YES

Close the 1st automatic valve — S17

Complete charging, separate charging nozzle — S18

Supplied gas amount-charged gas amount>Set value — S19    YES → Inform inspection results for pressure container — S20

Display charged amount, sum, safety — S21

NO

END

# FIG. 3

20

## INTERNATIONAL SEARCH REPORT

| International application No. |
|---|
| **PCT/KR2012/006589** |

### A. CLASSIFICATION OF SUBJECT MATTER

*F17C 13/02(2006.01)i, F17C 5/00(2006.01)i, B60K 15/06(2006.01)i*

According to International Patent Classification (IPC) or to both national classification and IPC

### B. FIELDS SEARCHED

Minimum documentation searched (classification system followed by classification symbols)
F17C 13/02; F17C 5/06; B60S 5/02

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: pressure container, charge, measurement

### C. DOCUMENTS CONSIDERED TO BE RELEVANT

| Category* | Citation of document, with indication, where appropriate, of the relevant passages | Relevant to claim No. |
|---|---|---|
| X<br>A | JP 07-186904 A (MASCHINENFABRIK SULZER-BURCKHARDT AG) 25 July 1995<br>See abstract, pages 1,2, claim 1. | 1-3<br>4,5 |
| A | JP 2002-206693 A (GREENFIELD AG) 26 July 2002<br>See abstract, pages 3-5, claim 1, figure 1. | 1-5 |
| A | JP 08-100887 A (TOKYO GAS CO LTD et al.) 16 April 1996<br>See abstract, claim 1, figure 1. | 1-5 |

☐ Further documents are listed in the continuation of Box C.    ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 21 JANUARY 2013 (21.01.2013) | **01 FEBRUARY 2013 (01.02.2013)** |

| Name and mailing address of the ISA/KR | Authorized officer |
|---|---|
| Korean Intellectual Property Office<br>Government Complex-Daejeon, 189 Seonsa-ro, Daejeon 302-701, Republic of Korea | |
| Facsimile No. 82-42-472-7140 | Telephone No. |

Form PCT/ISA/210 (second sheet) (July 2009)

**INTERNATIONAL SEARCH REPORT**
Information on patent family members

International application No.

**PCT/KR2012/006589**

| Patent document cited in search report | Publication date | Patent family member | Publication date |
|---|---|---|---|
| JP 07-186904 A | 25.07.1995 | AT 159803 T | 15.11.1997 |
| | | AU 677438 B2 | 24.04.1997 |
| | | AU 7764794 A | 18.05.1995 |
| | | BR 9404359 A | 04.07.1995 |
| | | CA 2135109 A1 | 09.05.1995 |
| | | CA 2135109 C | 07.04.1998 |
| | | DE 59404467 D1 | 04.12.1997 |
| | | EP 0653585 A1 | 17.05.1995 |
| | | EP 0653585 B1 | 29.10.1997 |
| | | JP 03-507148 B2 | 26.12.2003 |
| | | JP 3507148 B2 | 15.03.2004 |
| | | KR 10-0338885 B1 | 29.11.2002 |
| | | NZ 264820A | 24.10.1997 |
| | | US 05570729A A | 05.11.1996 |
| JP 2002-206693 A | 26.07.2002 | AR 031179A1 | 10.09.2003 |
| | | AU 2001-89243 A1 | 12.09.2002 |
| | | AU 2001-89243 B2 | 24.02.2005 |
| | | BR 0105077 A | 25.06.2002 |
| | | CA 2358583 A1 | 08.05.2002 |
| | | CA 2358583 C | 13.07.2004 |
| | | DE 50113779 D1 | 08.05.2008 |
| | | EP 1205704 A1 | 15.05.2002 |
| | | EP 1205704 B1 | 26.03.2008 |
| | | JP 04-030745 B2 | 09.01.2008 |
| | | US 2002-0053365 A1 | 09.05.2002 |
| | | US 6672340 B2 | 06.01.2004 |
| JP 08-100887 A | 16.04.1996 | NONE | |

Form PCT/ISA/210 (patent family annex) (July 2009)